# FASCICULE DE BREVET EUROPEEN

(11) **EP 0 486 091 B1**
(45) Date de publication et mention de la délivrance du brevet: **30.03.1994**
(21) Numéro de dépôt: 91202866.9
(22) Date de dépôt: 05.11.1991
(51) Int. Cl.: C07C 19/04, C07C 17/00, B01J 23/02

(54) **Procédé pour la fabrication du chloroforme à partir de tétrachlorure de carbone, compositions catalytiques et procédé pour leur obtention**
Verfahren zur Herstellung von Chloroform aus Kohlenstofftetrachlorid, katalytische Zusammensetzungen sowie Verfahren zu ihrer Herstellung
Process for the preparation of chloroform from carbon tetrachloride, catalytic compositions and process for their preparation

(30) Priorité: 16.11.1990 BE 9001086
(43) Date de publication de la demande: 20.05.1992
(73) Titulaire: SOLVAY (Société Anonyme), B-1050 Bruxelles (BE)
(72) Inventeur: Dogimont, Charles, B-1150 Bruxelles (BE); Franklin, James, B-1170 Bruxelles (BE); Janssens, Francine, B-1800 Vilvoorde (BE); Schoebrechts, Jean-Paul, B-1390 Grez-Doiceau (BE)
(74) Mandataire: Marckx, Frieda

(56) Documents cités:
- US-A- 3 579 596

## Description

L'invention concerne un procédé pour la fabrication du chloroforme à partir de tétrachlorure de carbone au moyen d'une composition catalytique. L'invention concerne également des compositions catalytiques particulières permettant un tel procédé.

Le brevet US 3,579,596 divulgue notamment des procédés de déchloration du tétrachlorure de carbone en chloroforme en phase gazeuse en présence d'un catalyseur choisi parmi les métaux Ru, Rh, Pd, Os, Ir, Pt, Cu, Ag ou Au et dont le support est formé par de l'alumine, du charbon actif ou de la silice.

Toutefois les procédés connus à ce jour sont accompagnés de réactions secondaires et/ou d'une désactivation rapide des catalyseurs, ce qui compromet l'efficacité de ces procédés.

L'invention, par contre, concerne un procédé de fabrication du chloroforme à partir du tétrachlorure de carbone qui ne présente plus ces inconvénients. On a en effet trouvé que certaines compositions catalytiques permettent une déchloration du tétrachlorure de carbone avec une sélectivité ou un taux de transformation, c'est-à-dire un rendement, qui n'ont jamais été atteints antérieurement, et plus particulièrement le procédé selon l'invention permet d'obtenir à partir de tétrachlorure de carbone presque exclusivement du chloroforme et du méthane, et de plus avec une formation réduite de composés en C₂.

Par ailleurs ces compositions catalytiques mises en oeuvre présentent principalement l'avantage d'être plus stables et de se désactiver beaucoup plus lentement que les compositions catalytiques connues.

L'invention concerne à cet effet un procédé de fabrication du chloroforme à partir du tétrachlorure de carbone en phase gazeuse en présence d'hydrogène moléculaire dans lequel la réaction est catalysée par une composition catalytique comportant au moins un métal à caractère hydrogénant déposé sur un support comprenant au moins un aluminate alcalin ou alcalino-terreux.

Par métal à caractère hydrogénant on entend les métaux du groupe VIII et du groupe IB du Tableau périodique des éléments. Habituellemment le métal hydrogénant est choisi parmi les métaux Ru, Rh, Pd, Os, Ir, Pt, Cu, Ag ou Au. De préférence le métal hydrogénant est le platine ou le palladium. De bons résultats ont été obtenus avec le platine.

Par support comprenant au moins un aluminate alcalin ou alcalino-terreux, on entend tous les supports catalytiques comprenant au moins un ou plusieurs aluminates d'un ou de plusieurs métaux alcalins ou alcalino-terreux. Généralement le support catalytique comprend au moins un aluminate alcalin. Habituellement le support catalytique comprend un aluminate de lithium, de magnésium ou de calcium. De préférence, le support comprend un aluminate présentant une structure d'oxyde mixte. De toute préférence, le support comprend un aluminate présentant une structure de spinelle inverse. De manière particulièrement préférée, le support comprend l'aluminate de lithium de formule LiAl₅O₈. De manière tout particulièrement préférée, le support comprend l'aluminate de lithium de formule LiAl₅O₈ et l'oxyde LiAlO₂. De bons résultats ont été obtenus avec une composition catalytique dont le support est constitué d'au moins 75 % d'aluminate LiAl₅O₈.

Le support catalytique peut aussi comprendre un aluminate mixte ou un mélange d'au moins deux aluminates de un ou de plusieurs métaux choisis parmi les métaux alcalins et les métaux alcalino-terreux. De bons résultats ont été obtenus avec une composition catalytique dans laquelle le support est un aluminate mixte de lithium et de sodium, de formule globale (Li,Na)₁Al₅O₈.

Généralement, on met en oeuvre de 0,01 à 5 % en poids de métal à caractère hydrogénant par rapport au poids de support de la composition catalytique. De préférence, on met en oeuvre de 0,05 à 1,0 % en poids. De bons résultats ont été obtenus avec de 0,2 à 0,6 % en poids de platine.

Le procédé selon l'invention a lieu en phase gazeuse.

La température à laquelle s'effectue la réaction, se situe habituellement entre 50 et 200°C. De préférence, cette température est comprise entre 80 et 170°C. De bons résultats ont été obtenus pour la fabrication sélective de chloroforme à partir du tétrachlorure de carbone avec une température réactionnelle située entre 90 et 150°C.

La pression à laquelle est effectuée la réaction, se situe généralement entre 0,1 et 10 ata. De préférence, elle se situe entre 1 et 8 ata. De bons résultats ont été obtenus avec une pression comprise entre 1 et 6 ata.

Le rapport molaire entre l'hydrogène et le tétrachlorure de carbone mis en oeuvre est généralement compris entre 1 et 16. De préférence, ce rapport est compris entre 4 et 14. De bons résultats ont été obtenus avec un rapport compris entre 6 et 12. Des rapports molaires situés dans cette plage favorisent la sélectivité en chloroforme, de plus la dilution de la phase gazeuse ralentit la formation de dépôts carbonés sur la composition catalytique.

Dans le même sens, la phase gazeuse est également avantageusement diluée par du méthane qui ralentit le vieillissement de la composition catalytique. Ainsi, le procédé selon l'invention peut être mis en oeuvre en présence de méthane. Généralement le rapport molaire entre le méthane et le tétrachlorure de carbone mis en oeuvre est compris entre 0,1 et 15. De préférence, ce rapport est compris entre 3 et 12. De bons résultats ont été obtenus avec un rapport compris entre 4 et 10.

Le temps de contact moyen, c'est-à-dire le rapport entre le volume de la partie du réacteur occupée par le catalyseur et le débit des réactifs à la pression et à la température de la réaction, est généralement compris entre 1 et 30 s. Habituellement il est compris entre 2 et 15 s. De bons résultats ont été obtenus avec un temps de contact compris entre 4 et 10 s.

On réalise le procédé selon l'invention avec une composition catalytique disposée en lit fixe ou en lit fluidisé.

On réalise le procédé selon l'invention dans tout appareillage ou réacteur permettant de réunir les conditions de réaction décrites.

L'invention concerne également des compositions catalytiques particulières pour la fabrication du chloroforme à partir du tétrachlorure de carbone.

L'invention concerne des compositions catalytiques comportant un support comprenant au moins un aluminate présentant une structure de spinelle inverse, sur lequel est déposé, comme métal à caractère hydrogénant, au moins du platine.

Le support des compositions catalytiques selon l'invention comprend au moins un aluminate présentant une structure de spinelle inverse. De préférence le support comprend l'aluminate de lithium de formule LiAl₅O₈. De manière particulièrement préférée le support comprend l'aluminate de lithium de formule LiAl₅O₈ et l'oxyde LiAlO₂. De bons résultats ont été obtenus avec une composition catalytique dont le support est constitué d'au moins 75 % d'aluminate LiAl₅O₈. Le support des compositions catalytiques peut aussi comprendre un aluminate mixte ou un mélange d'au moins deux aluminates d'un ou de plusieurs métaux choisis parmi les métaux alcalins et les métaux alcalino-terreux. De bons résultats ont été obtenus avec une composition catalytique dans laquelle le support est un aluminate mixte de lithium et de sodium de formule globale (Li,Na)₁Al₅O₈.

Les compositions catalytiques selon l'invention comprennent généralement de 0,01 à 5 % en poids de platine par rapport au poids de support de la composition catalytique. De préférence elles comprennent de 0,05 à 1,0 % en poids de platine par rapport au poids de support de la composition catalytique.

La surface spécifique des compositions catalytiques selon l'invention est généralement comprise entre 15 et 60 m²/g et de préférence entre 20 et 50 m²/g.

Le volume poreux des compositions catalytiques selon l'invention est généralement compris entre 0,1 et 0,8 cm³/g et de préférence entre 0,3 et 0,5 cm³/g.

Lorsque l'aluminate de lithium est le seul aluminate dans le support, le rapport Li/Al des compositions catalytiques selon l'invention est généralement compris entre 1/4 et 1/5,5 et de préférence est égal à environ 1/5.

Les compositions catalytiques peuvent être obtenues par tout procédé connu dans ce domaine.

Habituellement le procédé de préparation de ces compositions catalytiques comporte trois étapes :
- la préparation du support,
- l'imprégnation de ce support avec un composé du métal hydrogénant tel qu'un composé du platine et
- l'activation de la composition catalytique.

Un mode d'obtention du support compris dans les compositions catalytiques selon l'invention ayant donné de bons résultats consiste à mettre en contact une alumine et une solution d'un composé alcalin ou alcalino-terreux susceptible de former un oxyde, à éliminer l'eau puis à soumettre le produit obtenu à un cycle thermique de calcination qui transforme le composé alcalin ou alcalino-terreux en un oxyde correspondant et provoque la réaction entre les oxydes d'alumine et alcalins ou alcalino-terreux. D'excellents résultats ont été obtenus lorsqu'on met en contact une alumine gamma et un composé de lithium précurseur de l'oxyde tel qu'un nitrate ou un acétate.

Pour la préparation de la composition catalytique, la préparation du support est suivie de l'imprégnation du support avec un composé du métal hydrogénant tel qu'un composé du platine. Une préparation ayant donné de bons résultats consiste à mettre en contact le support et une solution acide d'un composé du platine sous vide. De préférence, on opère en présence d'acide chloroplatinique hexahydraté dissous dans de l'acide chlorhydrique.

Avant l'emploi, la composition catalytique est activée. Une activation qui a donné de bons résultats consiste à balayer la composition catalytique successivement :
- sous air ou sous oxygène à une température comprise entre environ 100 et 150°C,
- puis sous air ou sous oxygène à une température comprise entre environ 500 et 550°C,
- sous gaz inerte tel que l'azote, à une température comprise entre 75 et 125°C,
- sous hydrogène, à une température comprise entre 250 et 300°C et
- enfin sous gaz inerte tel que l'azote à température ambiante.

Après utilisation des compositions catalytiques de l'invention, la composition catalytique peut être régénérée sans perte importante d'activité et de sélectivité. Un procédé de régénération ayant donné de bons résultats consiste à appliquer à la composition catalytique deux traitements (balayage) par l'air à environ 350°C d'une durée de 24 h, chacun suivi d'une réduction sous hydrogène à environ 280°C pendant 8 heures. Un autre procédé consiste en une régénération sous hydrogène à une température comprise entre 200 et 350°C.

L'invention se trouve plus amplement illustrée par les exemples suivants.

### Exemple 1 - Préparation de la composition catalytique

### a) Préparation du support

Dans un erlenmeyer on introduit 16,4 g de LiOH.H₂O et 28 ml d'HNO₃ concentré. A la solution obtenue on ajoute de l'eau de façon à obtenir un volume total égal à 35 ml.

On sèche de l'alumine (alumine gamma de surface spécifique égale à 303 m²/g et de volume poreux égal à 0,35 cm³/g) à 100°C sous vide.

Puis on imprègne à température ambiante et à pression atmosphérique, 100 g d'alumine ainsi séchée avec la solution obtenue ci-dessus.

On laisse reposer une heure à la température ambiante.

Le produit ainsi obtenu est séché sous vide à 90°C durant 45 minutes.

Le support obtenu est ensuite calciné sous air par paliers de 6 heures à 600°C et 8 heures à 1050°C.

### b) Imprégnation

On dissout 5 g d'acide chloroplatinique hexahydraté dans 100 ml d'une solution d'acide chlorhydrique 0,05 M.

On prélève 4,5 ml de cette solution et on la dilue par 4,8 ml d'eau.

On place 17,1 g du support obtenu comme décrit au paragraphe a) pendant 2 heures sous vide à 100°C dans un ballon rainuré de 500 ml. On le laisse revenir à température ambiante sous vide. Puis on ajoute à ce support la solution, préparée ci-dessus, en 75 minutes et sous vide à température ambiante.

On laisse reposer la composition obtenue à température ambiante et à pression atmosphérique durant 24 heures.

On met la composition dans une étuve sous vide à 100°C pendant 72 heures.

### c) Activation

On place la composition obtenue comme décrit au paragraphe b) dans un réacteur pour calcination que l'on balaie sous air à 130°C pendant 4 heures.

On porte ensuite le réacteur à 530°C pendant 1 heure sous air.

Puis on refroidit le réacteur sous azote à 100°C et on le laisse dans ces conditions pendant une nuit.

Ensuite on porte le réacteur à 280°C sous azote et on le balaie ensuite sous hydrogène pendant 8 heures à 280°C.

On ramène le réacteur à température ambiante sous balayage d'azote.

Le rapport Li/Al de la composition catalytique est de 1/5. L'analyse par diffraction aux rayons X de la composition catalytique après broyage indique la présence de trois phases dont le constituant majoritaire est le LiAl₅O₈.

La surface spécifique et le volume poreux de la composition catalytique déterminés par adsorption d'azote sont respectivement de 33 m²/g et de 0,37 cm³/g.

### Exemple 2 - Fabrication du chloroforme à partir du tétrachlorure de carbone

10 cm³ (7,65 g) de la composition catalytique obtenue comme décrit à l'exemple 1 sont introduits en lit fixe dans un réacteur en acier inoxydable de diamètre intérieur de 10,2 mm et chauffé au moyen d'un four à air pulsé.

On alimente le réacteur par un mélange gazeux constitué de 82,8 vol % d'hydrogène et 17,2 vol % de tétrachlorure de carbone à une température de 105°C sous une pression de 4,3 bar et à une vitesse spatiale (c'est-à-dire l'inverse du temps du contact moyen) de 480 h⁻¹, ce qui correspond à un débit d'hydrogène de 12,3 lN/h et un débit de tétrachlorure de carbone de 2,56 lN/h.

Le taux de conversion initial du tétrachlorure de carbone est de 95 mol %.

Les produits formés sont le chloroforme, le dichlorométhane, le méthane, l'acide chlorhydrique et des traces d'éthane et de perchloroéthylène. Les sélectivités, basées sur le tétrachlorure de carbone converti, sont respectivement pour le chloroforme de 75 mol %, pour le dichlorométhane de 2 mol % et pour le méthane de 22 mol %.

Après passage de 152 kg de tétrachlorure de carbone/g de platine, c'est-à-dire après environ 330 heures de fonctionnement, le taux de conversion du tétrachlorure de carbone est encore de 81 mol %, les sélectivités des produits formés sont inchangées.

### Exemple 3 - Fabrication du chloroforme à partir du tétrachlorure de carbone - Dilution du tétrachlorure de carbone par l'hydrogène

Après 330 heures de fonctionnement du réacteur contenant la composition catalytique de l'exemple 1 dans les conditions de l'exemple 2, on introduit dans le réacteur un mélange gazeux constitué de 89,4 vol % d'hydrogène et 10,6 vol % de tétrachlorure de carbone à une température de 105°C sous une pression de 4 bar et à une vitesse spatiale de 480 h⁻¹, ce qui correspond à des débits d'hydrogène et de tétrachlorure de carbone respectivement de 12,40 et 1,47 lN/h.

Dans ces conditions le taux de conversion du tétrachlorure de carbone est de 84 mol %. Les sélectivités, basées sur le tétrachlorure de carbone converti, valent respectivement pour le chloroforme 84 mol %, pour le dichlorométhane 0,7 mol % et pour le méthane 15 mol %.

Après passage de 39 kg supplémentaires de tétrachlorure de carbone par g de platine, c'est-à-dire après environ 145 heures de fonctionnement supplémentaires, le taux de conversion du tétrachlorure de carbone est de 82 mol %, les sélectivités des produits formés demeurant inchangées.

### Exemple 4 - Essai longue durée

30 cm³ (24 g) de la composition catalytique obtenue à l'exemple 1 sont mélangés avec 20 cm³ de billes de verre, ce mélange est introduit en lit fixe dans un réacteur en acier inoxydable de diamètre intérieur de 20 mm, chauffé au moyen d'une circulation d'huile dans une double enveloppe.

Un mélange gazeux constitué de 44,4 vol % d'hydrogène, de 44,4 vol % de méthane et 11,2 vol % de tétrachlorure de carbone est envoyé au travers de la composition catalytique à une température de 102°C, sous une pression de 4,0 bar et à une vitesse spatiale de 514 h⁻¹ (par rapport à la composition catalytique non diluée), ce qui correspond à des débits d'hydrogène, de méthane et de tétrachlorure de carbone, respectivement de 20,0, 20,0 et 5,0 lN/h.

Le taux de conversion du tétrachlorure de carbone est de 98 mol %. Les principaux produits formés sont le chloroforme, le dichlorométhane, le méthane et l'acide chlorhydrique. Les sélectivités, basées sur le tétrachlorure de carbone converti, sont respectivement pour le chloroforme de 83 mol %, pour le dichlorométhane de 1 mol % et pour le méthane de 16 mol %.

Après passage de 270 kg de tétrachlorure de carbone par g de platine (environ 940 heures de fonctionnement), le taux de conversion du tétrachlorure de carbone et les sélectivités des produits formés demeurent inchangées.

### Exemple 5R (de comparaison)

10 cm³ (4,99 g) d'une composition catalytique contenant 0,5 % de platine déposée sur une alumine thêta, sont introduits en lit fixe dans un réacteur en acier inoxydable de diamètre intérieur de 10,2 mm, chauffé au moyen d'un four à air pulsé.

On alimente le réacteur par un mélange gazeux constitué de 83,1 vol % d'hydrogène et 16,9 vol % de tétrachlorure de carbone à une température de 90°C, sous une pression de 4,04 bar et à une vitesse spatiale de 514 h⁻¹, ce qui correspond à un débit d'hydrogène de 12,98 lN/h et un débit de tétrachlorure de carbone de 2,65 lN/h.

Le taux de conversion du tétrachlorure de carbone est de 90 mol %.

Les principaux produits formés sont le chloroforme, le dichlorométhane, le méthane, l'acide chlorhydrique, le perchloroéthylène et l'hexachloroéthane. Les sélectivités, basées sur le tétrachlorure de carbone converti, sont respectivement pour le chloroforme de 67 mol %, pour le dichlorométhane de 4 mol %, pour le méthane de 23 mol %, pour le perchloroéthylène de 5 mol % et pour l'hexachloroéthane de 0,5 mol %.

Après passage de 17 kg de tétrachlorure de carbone/g de platine, c'est-à-dire après environ 24 heures de fonctionnement, le taux de conversion du tétrachlorure de carbone est encore de 66 mol %, les sélectivités, basées sur le tétrachlorure de carbone converti, sont respectivement pour le chloroforme de 72 mol %, pour le dichlorométhane de 0 mol %, pour le méthane de 23 mol %, pour le perchloroéthylène de 3 mol % et pour l'hexachloroéthane de 2 mol %.

### Exemple 6 - Préparation de la composition catalytique

### a) Préparation du support

Dans un erlenmeyer, on introduit 8,2 g de LiOH.H₂O, 14 ml d'HNO₃ concentré et 16,62 g de NaNO₃. A la solution obtenue, on ajoute de l'eau de façon à obtenir un volume total égal à 44 ml.

On sèche de l'alumine (alumine gamma de surface spécifique égale à 300 m²/g et de volume poreux égal à 0,4 cm³/g) à 100°C sous vide.

Puis on imprègne à température ambiante et à pression atmosphérique, 100 g d'alumine ainsi séchée avec la solution obtenue ci-dessus.

On laisse reposer une heure à la température ambiante.

Le produit ainsi obtenu est séché sous vide à 90°C durant 45 minutes.

Le support obtenu est ensuite calciné sous air par paliers de 6 heures à 600°C et 8 heures à 1050°C.

La formule globale du support obtenu est (Li,Na)₁Al₅O₈ .

### b) Imprégnation

On dissout 5 g d'acide chloroplatinique hexahydraté dans 100 ml d'une solution d'acide chlorhydrique 0,05 M.

On prélève 26,5 ml de cette solution et on la dilue par 17,5 ml d'eau.

On place 100 g du support obtenu comme décrit au paragraphe a) pendant 45 minutes sous vide à 90°C dans un ballon rainuré de 500 ml. On le laisse revenir à température ambiante sous vide. Puis on ajoute à ce support la solution, préparée ci-dessus, en 80 minutes et sous vide à température ambiante.

On met la composition dans une étuve sous vide à 100°C pendant 24 heures.

### c) Activation

On place la composition obtenue comme décrit au paragraphe b) dans un réacteur pour calcination que l'on balaie sous air à 130°C pendant 4 heures.

On porte ensuite le réacteur à 530°C pendant 1 heure sous air.

Puis on refroidit le réacteur sous azote jusqu'à température ambiante et on le laisse dans ces conditions pendant une nuit.

Ensuite on porte le réacteur à 280°C sous azote et on le balaie ensuite sous hydrogène pendant 4 heures à 280°C.

On ramène le réacteur à température ambiante sous balayage d'azote.

La surface spécifique et le volume poreux de la composition catalytique determinés par adsorption d'azote sont respectivement de 45 m²/g et de 0,34 cm³/g.

### Exemple 7 - Fabrication du chloroforme à partir du tétrachlorure de carbone

10 cm³ (8,24 g) de la composition catalytique obtenue comme décrit à l'exemple 6 sont introduits en lit fixe dans un réacteur en acier inoxydable de diamètre intérieur de 10,2 mm et chauffé au moyen d'un four à air pulsé.

On alimente le réacteur par un mélange gazeux constitué de 83,3 vol % d'hydrogène et 16,7 vol % de tétrachlorure de carbone à une température de 90°C sous une pression de 4,0 bar et à une vitesse spatiale de 514 h⁻¹, ce qui correspond à un débit d'hydrogène de 12,9 lN/h et un débit de tétrachlorure de carbone de 2,6 lN/h.

Le taux de conversion initial du tétrachlorure de carbone est de 98 mol %.

Les produits formés sont le chloroforme, le dichlorométhane, le méthane, l'acide chlorhydrique et des traces d'éthane et de perchloroéthylène. Les sélectivités, basées sur le tétrachlorure de carbone converti, sont respectivement pour le chloroforme de 80 mol %, pour le dichlorométhane de 2 mol % et pour le méthane de 18 mol %.

### Exemple 8 - Fabrication du chloroforme à partir du tétrachlorure de carbone - Dilution du tétrachlorure de carbone par l'hydrogène ou par l'hydrogène et le méthane

Après 22 heures de fonctionnement du réacteur contenant la composition catalytique de l'exemple 6 dans les conditions de l'exemple 7, on introduit dans le réacteur un mélange gazeux constitué de 88,9 vol % d'hydrogène et 11,1 vol % de tétrachlorure de carbone à une température de 90°C sous une pression de 4 bar et à une vitesse spatiale de 514 h⁻¹, ce qui correspond à des débits d'hydrogène et de tétrachlorure de carbone respectivement de 13,7 et 1,7 lN/h.

Dans ces conditions, le taux de conversion du tétrachlorure de carbone est de 98 mol %. Les sélectivités, basées sur le tétrachlorure de carbone converti, valent respectivement pour le chloroforme 87 mol %, pour le dichlorométhane 1 mol % et pour le méthane 12 mol %.

Après 6 heures de fonctionnement dans ces conditions, on introduit dans le réacteur un mélange gazeux constitué de 44,4 vol % d'hydrogène et 44,4 vol % de méthane et 11,2 vol % de tétrachlorure de carbone à une température de 100°C sous une pression de 4 bar et à une vitesse spatiale de 514 h⁻¹, ce qui correspond à des débits d'hydrogène, de méthane et de tétrachlorure de carbone respectivement de 6,7 lN/h, 6,7 lN/h et 1,7 lN/h.

Dans ces conditions, le taux de conversion du tétrachlorure de carbone est de 98 mol %. Les sélectivités, basées sur le tétrachlorure de carbone converti, valent respectivement pour le chloroforme 86 mol %, pour le dichlorométhane 1 mol % et pour le méthane 13 mol %.

## Revendications

1. Procédé pour la fabrication du chloroforme à partir du tétrachlorure de carbone en phase gazeuse en présence d'hydrogène moléculaire dans lequel la réaction est catalysée par une composition catalytique caractérisé en ce que la composition catalytique comporte au moins un métal à caractère hydrogénant déposé sur un support comprenant au moins un aluminate alcalin ou alcalino-terreux.

2. Procédé selon la revendication 1 caractérisé en ce que le support comprend au moins un aluminate présentant une structure de spinelle inverse.

3. Procédé selon une quelconque des revendications 1 ou 2, caractérisé en ce que le support comprend un aluminate de lithium de formule LiAl₅O₈.

4. Procédé selon une quelconque des revendications 1 à 3, caractérisé en ce que le support comprend un aluminate mixte d'au moins deux métaux choisis parmis les métaux alcalins et les métaux alcalino-terreux.

5. Procédé selon une quelconque des revendications 1 à 3, caractérisé en ce que le support comprend un mélange d'au moins deux aluminates de métaux choisis parmis les métaux alcalins et les métaux alcalino-terreux.

6. Procédé selon une quelconque des revendications 1 à 5, caractérisé en ce que le rapport molaire entre l'hydrogène et le tétrachlorure de carbone est compris entre 1 et 16.

7. Procédé selon une quelconque des revendications 1 à 6, caractérisé en ce que la phase gazeuse est diluée par du méthane.

8. Procédé selon une quelconque des revendications 1 à 7, caractérisé en ce que la température à laquelle s'effectue la réaction, est comprise entre 50 et 200°C.

9. Compositions catalytiques comportant un support sur lequel est déposé au moins du platine caractérisées en ce que le support comprend au moins un aluminate présentant une structure de spinelle inverse.

10. Compositions catalytiques selon la revendication 9 caractérisées en ce que le support comprend un aluminate de lithium de formule LiAl₅O₈.

11. Compositions catalytiques selon la revendication 9 caractérisées en ce que le support comprend un aluminate mixte de lithium et de sodium, de formule (Li,Na)₁Al₅O₈.

## Patentansprüche

1. Verfahren zur Herstellung von Chloroform aus Kohlenstofftetrachlorid in gasförmiger Phase in Anwesenheit von molekularem Wasserstoff, bei dem die Reaktion durch eine katalytische Zusammensetzung katalysiert wird, dadurch gekennzeichnet, daß die katalytische Zusammensetzungwenigstens ein Metall mit hydrierenden Eigenschaften umfaßt, das auf einem Träger abgelagert ist, der wenigstens ein Alkali- oder Erdalkalialuminat umfaßt.

2. Verfahren nach Anspruch 1, dadurch gekennzeichnet, daß der Träger wenigstens ein eine inverse Spinell-Struktur aufweisendes Aluminat umfaßt.

3. Verfahren nach einem der Ansprüche 1 oder 2, dadurch gekennzeichnet, daß der Träger ein Lithiumaluminat der Formel LiAl₅O₈ umfaßt.

4. Verfahren nach einem der Ansprüche 1 bis 3, dadurch gekennzeichnet, daß der Träger ein Aluminat, gemischt aus wenigstens zwei Metallen, ausgewählt unter den Alkalimetallen und den Erdalkalimetallen, umfaßt.

5. Verfahren nach einem der Ansprüche 1 bis 3, dadurch gekennzeichnet, daß der Träger eine Mischung von wenigstens zwei Aluminaten von Metallen, ausgewählt unter den Alkalimetallen und den Erdalkalimetallen umfaßt.

6. Verfahren nach einem der Ansprüche 1 bis 5, dadurch gekennzeichnet, daß das molare Verhältnis zwischen dem Wasserstoff und dem Kohlenstofftetrachlorid zwischen 1 und 16 beträgt.

7. Verfahren nach einem der Ansprüche 1 bis 6, dadurch gekennzeichnet, daß die gasförmige Phase durch Methan verdünnt wird.

8. Verfahren nach einem der Ansprüche 1 bis 7, dadurch gekennzeichnet, daß die Temperatur, bei der die Reaktion durchgeführt wird, zwischen 50 und 200°C beträgt.

9. Katalytische Zusammensetzungen, umfassend einen Träger, auf dem wenigstens Platin abgelagert ist, dadurch gekennzeichnet, daß der Träger wenigstens ein Aluminat umfaßt, das eine inverse Spinell-Struktur aufweist.

10. Katalytische Zusammensetzungen nach Anspruch 9, dadurch gekennzeichnet, daß der Träger ein Lithiumaluminat der Formel LiAl₅O₈ umfaßt.

11. Katalytische Zusammensetzungen nach Anspruch 9, dadurch gekennzeichnet, daß der Träger ein gemischtes Aluminat aus Lithium und Natrium, der Formel (Li,Na)₁Al₅O₈, umfaßt.

## Claims

1. Process for the production of chloroform from carbon tetrachloride in the gas phase in the presence of molecular hydrogen, in which the reaction is catalysed by a catalytic composition, characterized in that the catalytic composition comprises at least one metal having a hydrogenating character deposited on a support comprising at least one alkali metal aluminate or alkaline-earth metal aluminate.

2. Process according to Claim 1, characterized in that the support comprises at least one aluminate exhibiting an inverse spinel structure.

3. Process according to either of Claims 1 and 2, characterized in that the support comprises a lithium aluminate of formula LiAl₅O₈.

4. Process according to any one of Claims 1 to 3, characterized in that the support comprises a mixed aluminate of at least two metals chosen from the alkali metals and the alkaline-earth metals.

5. Process according to any one of Claims 1 to 3, characterized in that the support comprises a mixture of at least two aluminates of metals chosen from the alkali metals and the alkaline-earth metals.

6. Process according to any one of Claims 1 to 5, characterized in that the molar ratio of hydrogen to carbon tetrachloride is between 1 and 16.

7. Process according to any one of Claims 1 to 6, characterized in that the gas phase is diluted with methane.

8. Process according to any one of Claims 1 to 7, characterized in that the temperature at which the reaction takes place is between 50 and 200°C.

9. Catalytic compositions comprising a support on which at least platinum is deposited, characterized in that the support comprises at least one aluminate exhibiting an inverse spinel structure.

10. Catalytic compositions according to Claim 9, characterized in that the support comprises a lithium aluminate of formula LiAl₅O₈.

11. Catalytic compositions according to Claim 9, characterized in that the support comprises a mixed lithium sodium aluminate, of formula (Li,Na)₁Al₅O₈.
